(19) Europäisches Patentamt
European Patent Office
Office européen des brevets

(11) **EP 2 265 323 B1**

(12) **EUROPEAN PATENT SPECIFICATION**

(45) Date of publication and mention
of the grant of the patent:
**07.09.2016 Bulletin 2016/36**

(21) Application number: **09721695.6**

(22) Date of filing: **13.03.2009**

(51) Int Cl.:
**A61N 1/05** (2006.01)

(86) International application number:
**PCT/US2009/001594**

(87) International publication number:
**WO 2009/117069 (24.09.2009 Gazette 2009/39)**

(54) **LOW PROFILE MEDICAL DEVICES WITH INTERNAL DRIVE SHAFTS THAT COOPERATE WITH RELEASABLY ENGAGEABLE DRIVE TOOLS**

MEDIZINISCHE NIEDRIGPROFILINSTRUMENTE MIT INTERNEN ANTRIEBSWELLEN IN KOPPELUNG MIT LÖSBAREN EINSCHALTBAREN ANTRIEBSWERKZEUGEN

DISPOSITIFS MÉDICAUX À FAIBLE PROFIL COMPORTANT DES AXES D'ENTRAÎNEMENT INTERNES COOPÉRANT AVEC DES INSTRUMENTS D'INTRODUCTION AMOVIBLES

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **17.03.2008 US 37084 P**

(43) Date of publication of application:
**29.12.2010 Bulletin 2010/52**

(73) Proprietors:
• **Surgivision, Inc.**
**Memphis, TN 38103 (US)**
• **Boston Scientific Neuromodulation Corporation**
**Valencia, CA 91355 (US)**

(72) Inventor: **GORE, Brian**
**Ormond Beach**
**Florida 32174-5623 (US)**

(74) Representative: **Vossius & Partner**
**Patentanwälte Rechtsanwälte mbB**
**Siebertstrasse 3**
**81675 München (DE)**

(56) References cited:
**US-A- 4 357 946          US-A1- 2003 204 232**
**US-A1- 2004 014 355    US-A1- 2004 014 355**
**US-A1- 2008 109 042    US-B2- 6 813 521**

**Description**

**BACKGROUND**

Medical leads can have active fixation ends that extend to engage local tissue during a surgical procedure such as placement of implantable leads in the body for cardiac pace-making. In the past, an inner conductor has been configured to rotate to extend the screw end out of the lead while applying torque. There remains a need for alternate designs that allow for low profile lead configurations.
US 2003/204232 A1 discloses an electrical medical lead, having two or more electrodes, electrically insulated from each other and electrically coupled to individually insulated filars in a multi-filar coiled conductor. One embodiment includes a tip electrode array that is expandable for improving electrode contact with targeted tissue and stabilizing lead position.

**SUMMARY OF EMBODIMENTS OF THE INVENTION**

Embodiments of the present invention are directed to medical leads with integral drive shafts that can rotate. The leads can be low-profile and flexible and may be MRI-safe.
According to the present invention, there is provided a medical device comprising:

an elongate body having opposing proximal and distal end portions with an axially extending center cavity;
an internal drive shaft residing in the center cavity, the drive shaft having a proximal end portion with a rotatable spline residing in the proximal end portion of the elongate body; and
an extendable member (80) held in the distal end portion of the elongate body (10), the extendable member in communication with the drive shaft, whereby rotation of the drive shaft causes the extendable member to extend out of the elongate body, characterised by at least one conductor (60) extending along the elongate body, each of the at least one conductor comprising at least one current suppression module (64), each of the at least one current suppression module comprising the conductor turning back on itself twice in a lengthwise direction to from a conductor configuration of a reverse or backward section in one lengthwise direction and proximately located forward sections that extend in an opposing lengthwise direction.

Preferably, the elongate body is an intrabody medical lead with at least one electrode with low DC resistance and is flexible.
Advantageously, the extendable member comprises a screw electrode.
Conveniently, the extendable member is an electrode or sensor in electrical communication with a one of the at least one conductor, and wherein the elongate body is an implantable lead and has a diameter that is less than about 0.10 inches (2.5 mm) over at least a major portion of its length.
Preferably, the elongate body is an implantable neuromodulation lead.
Conveniently, the elongate body is an implantable cardiac lead.
Advantageously, the elongate body is an implantable pacemaker lead.
Preferably, the elongate body comprises a flexible inner sleeve residing over the drive shaft and at least one conductor portion coiled substantially concentrically about the sleeve.
Advantageously, the elongate body comprises a stationary electrode disposed on the proximal end portion of the elongate body about the drive shaft spline.
Conveniently, the medical device further comprises a single-use disposable drive tool, the drive tool having a primary body with an axially extending cavity and a spline and/or spline engagement member residing in a distal end portion of drive the tool primary body, the tool spline and/or spline engagement member adapted to slidably releasably engage the drive shaft spline whereby a user can rotate the drive shaft.
Conveniently, the drive tool primary body has a bore sized and configured to snugly slidably receive the proximal end portion of the elongate body, wherein the bore has a larger diameter than the axially extending cavity.
Advantageously, the medical device and tool further comprise a stylet that extends out of the proximal end portion of the drive tool and resides in a center cavity extending through the drive shaft.
Preferably, the elongate body is an implantable pacemaker lead and the drive shaft is an internal rotatable and translatable drive shaft.
Advantageously, the lead has low DC resistance and is flexible, wherein the extendable member comprises a fixation screw, and wherein the lead is an active fixation bradyarrhythmia lead with a distal electrode.
Conveniently, the medical device further comprises a single-use disposable medical drive tool having an internal spline or spline engagement member sized and configured to slidably releasably receive and engage the rotatable spline residing in the proximal end portion of the elongate body, the drive tool further comprising a cavity for receiving a stylet, the drive tool being configured to allow a user to both rotate and translate the internal drive shaft, wherein the medical drive tool is held in a sterile package.

Further features, advantages and details of the present invention will be appreciated by those of ordinary skill in the art from a reading of the figures and the detailed description of the embodiments that follow, such description being merely illustrative of the present invention.

## DRAWINGS

Figure 1 is a partial cutaway, partial transparent side view of a lead having a driveshaft according to embodiments of the present invention.

Figure 2 is a partially transparent end perspective view of the lead shown in Figure 1.

Figure 3 is a sectional side view of the proximal end of the lead shown in Figure 1.

Figure 4A is a sectional side view of a drive tool according to embodiments of the present invention, illustrating the lead shown in Figure 1 aligned but not fully engaged according to embodiments of the present invention.

Figure 4B is a sectional side view of the device shown in Figure 4A, illustrating the lead shown in Figure 1 in operative position according to embodiments of the present invention.

Figure 5A is partial transparent and cutaway side view of the proximal end of the lead shown in Figure 1 with the drive shaft in a retracted configuration according to embodiments of the present invention.

Figure 5B is a partial transparent and cutaway side view of the device shown in Figure 5A with the drive shaft in an extended configuration according to embodiments of the present invention.

Figure 6 is a partial side sectional view of a portion of the lead shown in Figure 1, including the proximal portion.

Figure 7 is a partial side sectional and partially transparent view of the portion of the lead shown in Figure 6 illustrating additional features according to embodiments of the present invention.

Figure 8 is a partially transparent side view of the lead shown in Figure 1 with the distal portion shown according to embodiments of the present invention.

## DETAILED DESCRIPTION

The present invention now is described more fully hereinafter with reference to the accompanying drawings, in which embodiments of the invention are shown. This invention may, however, be embodied in many different forms and should not be construed as limited to the embodiments set forth herein; rather, these embodiments are provided so that this disclosure will be thorough and complete, and will fully convey the scope of the invention to those skilled in the art.

Like numbers refer to like elements throughout. In the figures, the thickness of certain lines, layers, components, elements or features may be exaggerated for clarity. Broken lines illustrate optional features or operations unless specified otherwise.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof. As used herein, the term "and/or" includes any and all combinations of one or more of the associated listed items. As used herein, phrases such as "between X and Y" and "between about X and Y" should be interpreted to include X and Y. As used herein, phrases such as "between about X and Y" mean "between about X and about Y." As used herein, phrases such as "from about X to Y" mean "from about X to about Y."

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the specification and should not be interpreted in an idealized or overly formal sense unless expressly so defined herein. Well-known functions or constructions may not be described in detail for brevity and/or clarity.

It will be understood that when an element is referred to as being "on", "attached" to, "connected" to, "coupled" with, "contacting", etc., another element, it can be directly on, attached to, connected to, coupled with or contacting the other element or intervening elements may also be present. In contrast, when an element is referred to as being, for example, "directly on", "directly attached" to, "directly connected" to, "directly coupled" with or "directly contacting" another element, there are no intervening elements present. It will also be appreciated by those of skill in the art that references to a structure or feature that is disposed "adjacent" another feature may have portions that overlap or underlie the adjacent feature.

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if the device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

It will be understood that, although the terms first, second, etc. may be used herein to describe various elements, components, regions, layers and/or sections, these elements, components, regions, layers and/or sections should not be limited by these terms. These terms are only used to distinguish one element, component, region, layer or section from another region, layer or section. Thus, a first element, component, region, layer or section discussed below could be termed a second element, component, region, layer or section without departing from the teachings of the present invention. The sequence of operations (or steps) is not limited to the order presented in the claims or figures unless specifically indicated otherwise. Certain of the figures illustrate the device as partially transparent (the affected components so shown indicated by broken lines) for ease of reference to internal components.

The term "drive shaft" refers to a rotating member that transmits torque or otherwise advances and/or retracts a target member. The term "spline" refers to a series of projections on a shaft that fit into slots or mating projections on a corresponding shaft, thereby allowing both to rotate together while one shaft translates relative to the other. Thus, one shaft can have a first spline and a second shaft can have a matably engaging spline or a spline engagement member. The spline or spline engagement member can comprise slots, projections and/or recesses and the like, that engage the target spline to allow both shafts to rotate together while translating relative to each other.

The term "lead" refers to an elongate assembly that includes one or more conductors. The lead typically connects two spaced apart components, such as, for example, a power source and/or input at one end portion and an electrode and/or sensor at another position, such as at a distal end portion, or electrodes at both end portions. The lead is typically flexible. The lead can be substantially tubular with a cylindrical shape, although other shapes may be used. The lead can have a solid or hollow body and may optionally include one or more lumens. In particular embodiments, a lead can be a relatively long implantable lead having a physical length of greater than about 10 cm (up to, for example, 1 m, or even longer). The lead can be an intrabody medical lead for acute or chronic use, including, for example, implantable leads. The lead can be for veterinary or human use.

The term "conductor" and derivatives thereof refer to a conductive trace, filar, wire, cable, flex circuit or other electrically conductive member. A conductor may also be configured as a closely spaced bundle of filars or wires. The conductor can be a single continuous length. The conductor can be formed with one or more of discrete filars, wires, cables, flex circuits, bifilars, quadrafilars or other filar or trace configuration, or by plating, etching, deposition, or other fabrication methods for forming conductive electrical paths. The conductor can be insulated. The conductor can also comprise any suitable MRI-compatible (and biocompatible) material such as, for example, MP35N drawn filled tubing with a silver core and an ETFE insulation on the drawn tubing.

The term "current suppression module" ("CSM") refers to an elongate conductor that turns back on itself at least twice in a lengthwise direction to form a conductor configuration of a reverse or backward section in one lengthwise direction and proximately located forward sections that extend in the opposing lengthwise direction. The CSM can be configured with a length that is a sub-length of the overall length of the conductor, e.g., less than a minor portion of the length of the conductor, and the conductor can have multiple CSMs along its length. The term "MCSM" refers to a conductor that has multiple CSMs, typically arranged at different locations along at least some, typically substantially all, of its length. The terms "backward", "rearward" and "reverse" and derivatives thereof are used interchangeably herein to refer to a lengthwise or longitudinal direction that is substantially opposite a forward lengthwise or longitudinal direction. The words "sections", "portions" and "segments" and derivatives thereof are also used interchangeably herein and refer to discrete sub-portions of a conductor or lead.

The term "MRI compatible" means that the material is selected so as to be non-ferromagnetic and to not cause MRI operational incompatibility, and may also be selected so as not to cause undue artifacts in MRI images. The term "RF safe" means that the device, lead or probe is configured to operate within accepted heat-related safety limits when exposed to normal RF signals associated with target (RF) frequencies such as those frequencies associated with conventional MRI systems or scanners.

The term "high impedance" means an impedance that is sufficiently high to reduce, inhibit, block and/or eliminate flow of RF-induced current at a target frequency range(s). The impedance has an associated resistance and reactance as is well known to those of skill in the art. Some embodiments of the lead and/or conductors of the instant invention may provide an impedance of at least about 100 Ohms, typically between about 400 Ohms to about 600 Ohms, such as between about 450 Ohms to about 500 Ohms, while other embodiments provide an impedance of between about 500 Ohms to about 1000 Ohms or higher.

Embodiments of the invention configure leads that are safe (heat-resistant) at frequencies associated with a plurality of different conventional and future magnetic field strengths of MRI systems, such as at least two of 0.7T, 1.0T, 1.5T, 2T, 3T, 7T, 9T, and the like, and that allow for safe use in those environments (future and reverse standard MRI Scanner system compatibility).

The term "tuned", with respect to a coil, means tuned to define a desired minimal impedance at a certain frequency band(s) such as those associated with one or more high-field MRI Scanner systems. When used with respect to a parallel resonant circuit with inductive and capacitive characteristics defined by certain components and configurations, the word "tuned" means that the circuit has a high impedance at one or more target frequencies or frequency bands, typically including one or more MRI operating frequencies.

The term "coiled segment" refers to a conductor (e.g., trace, wire or filar) that has a coiled configuration. The coil may have revolutions that have a substantially constant diameter or a varying diameter or combinations thereof. The term "co-wound segments" means that the affected conductors can be substantially concentrically coiled at the same or different radii, *e.g.,* at the same layer or one above the other. The term "co-wound" is used to describe structure indicating that more than one conductor resides closely spaced in the lead and is not limiting to how the structure is formed (*i.e.,* the coiled segments are not required to be wound concurrently or together, but may be so formed).

The term "revolutions" refers to the course of a conductor as it rotates about its longitudinal/lengthwise extending center axis. A conductor, where coiled, can have revolutions that have a substantially constant or a varying (radius) distance from its center axis or combinations of constant and varying distances for revolutions thereof.

The term "Specific Absorption Rate" (SAR) is a measure of the rate at which RF energy is absorbed by the body when exposed to radio-frequency electromagnetic fields. The SAR is a function of input power associated with a particular RF input source and the object exposed to it, and is typically measured in units of Watts per kilogram (W/kg) taken over volumes of 1 gram of tissue or averaged over ten grams of tissue or over the entire sample volume, or over the volume of the exposed portion of the sample. SAR can be expressed as a peak input and/or whole body average value. Different MRI Scanners may measure peak SAR in different ways, resulting in some variation as is well known to those of skill in the art, while whole body average values are typically more consistent between different MR Scanner manufacturers.

Peak input SAR measurement is an estimate of the maximum input RF energy deposited in tissue during an MRI scan. To measure peak SAR, the following methodology using a suitable phantom can be employed. The peak SAR temperature(s) is typically measured near the surface. The phantom can be any shape, size and/or volume and is typically substantially filled with a medium simulating tissue, *e.g.,* the medium has electrical conductivity corresponding to that of tissue-typically between about 0.1-1.0 siemens/meter. The medium can be a gel, slurry, or the like, as is well known, and has conduction and/or convective heat transfer mechanisms. Peak input SAR is estimated based on temperature rise measured by the sensors placed near the surface/sides of the phantom and is calculated by Equation 1 as stated below. *See also*, ASTM standard F2182-02A, which described a way to measure input SAR.

$$dT/dt = SAR/ C_p \qquad\qquad \text{Equation (1)}$$

where: dT is the temperature rise
dt is the change in time
$C_p$ is the constant pressure specific heat of water (approx. 4180 J/kg-°C).

The term "low DC resistance" refers to leads having less than about 1 Ohm/cm, typically less than about 0.7 Ohm/cm, so, for example, a 60-70 cm lead can have DC resistance that is less than 50 Ohms. In some embodiments, a lead that is 73 cm long can have a low DC resistance of about 49 Ohms. Low DC resistance can be particularly appropriate for leads that connect power sources to certain components, *e.g.,* electrodes and IPGs for promoting low-power usage and/or longer battery life.

The lead can have good flexibility and high fatigue resistance to allow for chronic implantation. For example, with respect to flexibility, the lead can easily bend over itself. In some embodiments, the lead, when held suspended in a medial location, is sufficiently flexible so that the opposing long segments drape or droop down together (do not hold a specific configuration).

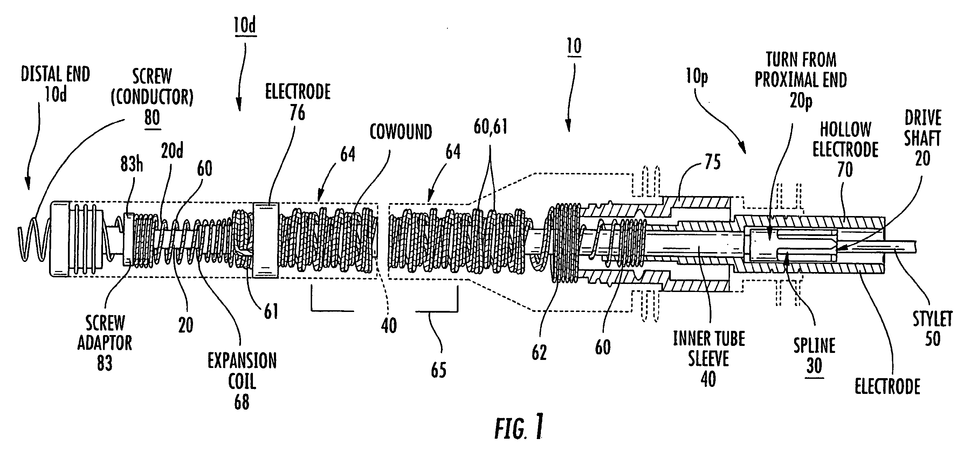

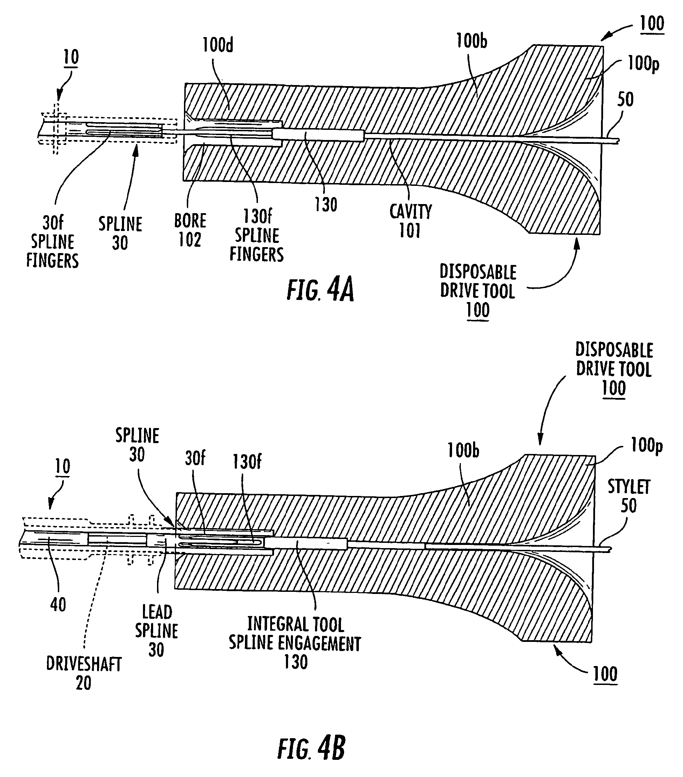

Turning now to the figures, **Figures 1-3** and **5-8** illustrate an exemplary lead **10** with opposing proximal and distal end portions **10p**, **10d**, respectively. The lead **10** has an internal drive shaft **20** extending from the proximal end portion **10p** to a distal end portion **10d**. The proximal end portion of the drive shaft **20p** can comprise a spline **30** or a spline engagement member that engages a spline of another releasably engageable shaft associated with a tool **100** **(Figures 4A, 4B)** used to position the lead in the body (*e.g.*, for acute internventional therapy or chronic implantation). The tool **100** can include an integral spline or spline engagement member **130 (Figures 4A, 4B)** that may cooperate with a stylet **50.** The spline shaft of the tool when engaged to the lead drive shaft **20** is used to deploy the extendable member. For example, a clinician can linearly translate then rotate the tool **100** thereby rotating the member **130,** which, in turn causes the drive shaft **20** of the lead to turn. The stylet **50** is optional but can provide additional rigidity to the lead during placement in the body. The distal end portion of the drive shaft **20d** is in communication with a deployable or extendable member **80** that can be advanced and, optionally, retracted, in response to translation and rotation of the drive shaft **20**. That is, clockwise or counterclockwise rotation of the drive shaft **20** can cause the target member **80** to rotate and advance out of the tip end of the lead (and, in some embodiments, rotation in the reverse direction can cause it to retract back into the tip or end of the lead).

As shown, the target extendable member is a screw **80.** The screw can comprise a conductor material and the screw **80** can be attached to a screw adaptor **83** with a hub **83h.** The screw **80** can also act as an electrode to transmit energy to local tissue. Rotation of the drive shaft **20** causes the screw conductor **80** to rotate linearly translate between about 1 mm to about 1 cm. The tip nut **90** has internal threads that mesh with the screw, causing the screw to extend or retract when it rotates, along with the driveshaft and spline. The term "screw" refers to a member having a pointed substantially rigid spiral or helical fixation screw such as a corkscrew-like configuration as shown in the exemplary extendable member. The expansion coil **68** may connect a lead to the screw, while allowing for the translation and rotation of the screw by winding up or unwinding during the process. Although shown as a screw **80,** the target extendable member **80** can be other members with other configurations, such as, for example, a needle, a sensor, a barb or anchor, a delivery device (drug or other therapy), a biopsy device, and the like.

The lead **10** can be a low profile lead with at least a major portion of its body having a cross-sectional area or diameter of about 0.20 inches (5.1 mm) or less. In some embodiments, the lead **10** is a low profile lead with a cross-sectional area or diameter that is between about 0.001 inches (0.03 mm) to about 0.085 inches (2.2 mm) over at least a portion of its length, *e.g.*, such as at least a distal end portion of the lead **10d**. In particular embodiments, the lead **10** can have a diameter or cross-sectional width or length that is between about 0.01 inches (0.3 mm) to about 0.18 inches (4.6 mm) over at least a major portion of its length, such as about 0.10 inches (2.5 mm).

**Figures 1-3** also illustrate that the lead **10** can have at least one electrode, shown as having three axially spaced apart electrodes, **70, 75, 76.** At least one conductor extends to each of the electrodes **75, 76.** The first electrode **70** can be a hollow electrode with a cavity that is sized and configured to receive the drive shaft **20** and spline **30.** The second electrode **75** can extend over the hollow electrode **70.** Each of the first and second electrodes **70, 75** can be fixed (*e.g.*, static and non-rotating). The second electrode **75** can be affixed to the first electrode body **70** via adhesive or overmolding or the like. The space **71** between the two electrodes **70, 75** can be insulated, such as with silicone during a molding or overmolding process. The first electrode **70** can be affixed to the sleeve **40,** which also is fixed (*e.g.,* static and non-rotating). The drive shaft **20** and its proximal end **20p** move or translate with respect to the sleeve and electrodes **70, 75.**

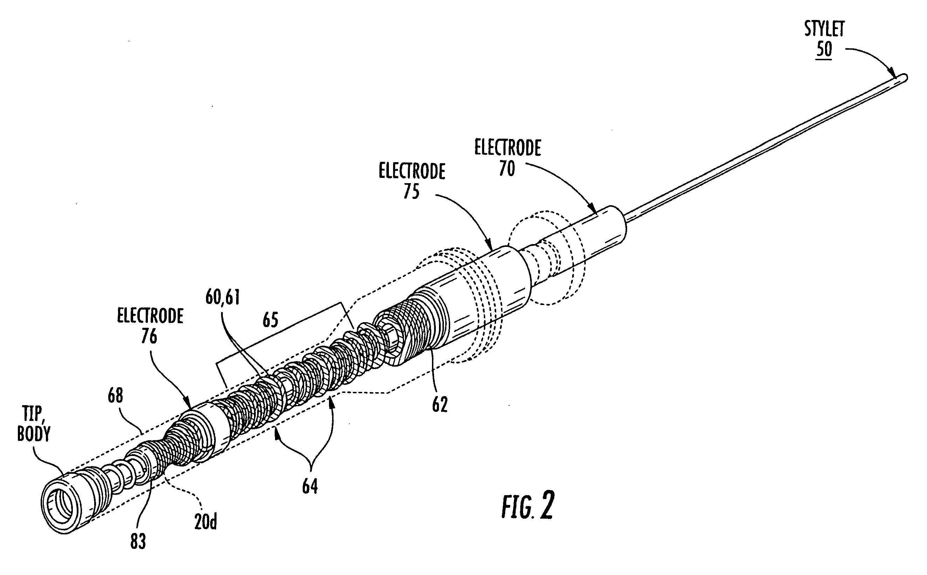

The expansion coil **68** can be defined by an extension or continuation of one or more of the conductors, shown as the inner conductor **60.** Each conductor can include at least one CSM **64,** shown as MCSMs **65** along their length as shown in **Figures 1** and **2.** The lead **10** can include two inner conductors **60, 61** that are cowound and define stacked (multi-layer coils) which are substantially concentric and turn lengthwise directions at least twice to form the CSMs **64.** One of the conductors **60** can extend beyond the electrode **76** to form the expansion coil **68** and electrically connect the screw adaptor **83.** The other conductor **61** terminates proximate the electrode **76.**

The two inner conductors **60, 61** can reside over an inner flexible sleeve **40** as also shown in **Figures 1-3.** The inner conductors **60, 61** can be substantially concentric. The sleeve **40** can be static and be sized and configured to receive the drive shaft **20.** The sleeve **40** can terminate in advance of the screw adaptor **83.** For a discussion of fabrication methods and two and three-layer coil stacked coil configurations of one or more conductors, *see,* co-pending U.S. Patent Application Serial Nos. 60/955,724 and 12/047,832, the contents of each of which are hereby incorporated by reference as if recited in full herein.

**Figures 4A** and **4B** illustrate that the lead **10** can be slidably advanced into a distal end **100d** of the tool body **100b.** The tool body **100b** can have a through cavity **101** which merges into a larger bore **102** containing fixed spline **130,** that snugly and slidably receives and releasably engages the spline or spline engagement member **30** of the lead **10.** The bore **102** can terminate into a stop position for secure engagement. The bore may have a countersunk lead-in edge to faciliate self-alignment. The drive tool **100** can be single-use disposable. A medical kit can be provided with a spare drive tool **100** in a sterile package for future use or the drive tool can be provided as a separate component so that a clinician can readily access the drive tool for future adjustment of the lead as appropriate (not shown). The tool body **100b** can be ergonomically configured to allow a clinician to hold as a hand, finger or thumb tool for precisely advancing the extendable member. The tool body **100b** and the lead can be MRI compatible and indeed, the tool body can be used to implant the lead during an MRI interventional procedure.

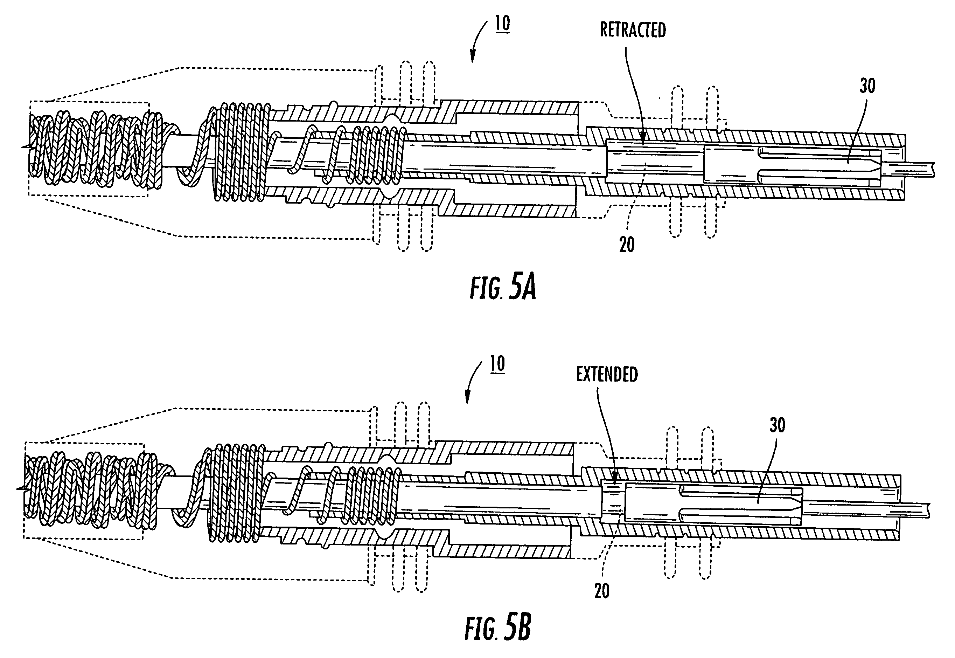

**Figures 5A** and **5B** illustrate exemplary retracted and extended configurations of the drive shaft **30** in the lead **10,** respectively. As shown in **Figure 5B**, the drive shaft **20** can have a linear stroke distance **"L"** of suitable distance, such as, for example, between about 0.1 mm to about 1 cm.

**Figures 4A** and **4B** also illustrate that both the lead **10** and tool **100** can include respective splines **30,130** with each including a series of forwardly projecting fingers **30f**, **130f** that slide together to matably engage and allow the drive shaft **20** to rotate while extending or retracting.

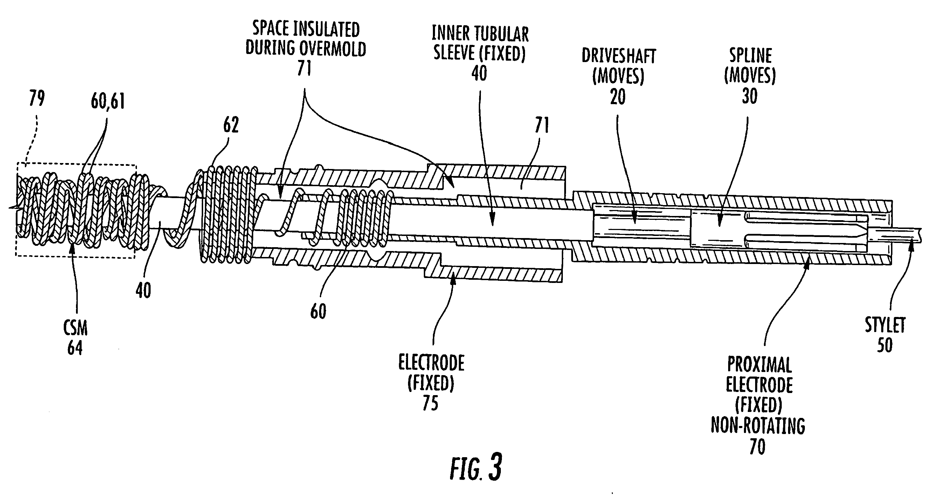

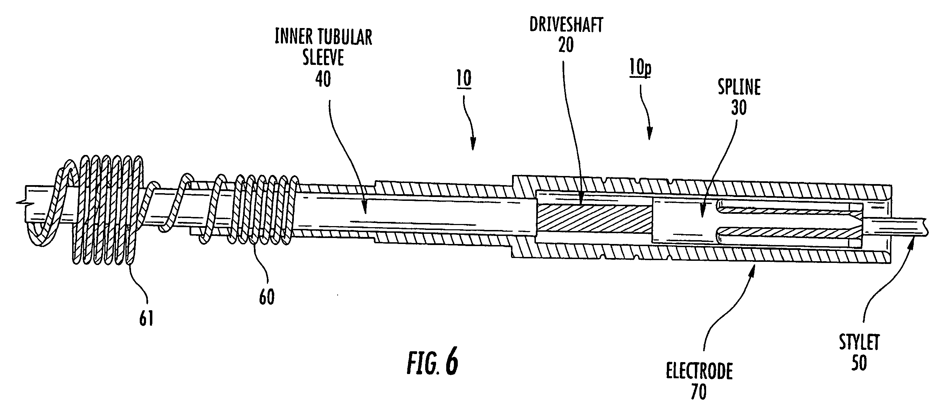

**Figures 3** and **6** illustrate that the drive shaft **20** can comprise a substantially rigid polymer such as, for example, polyimide. The drive shaft **20** can have an inner diameter of less than about 0.028 inches (0.71 mm), such as about 0.018 inches (0.46 mm) and an outer diameter of less than about 0.024 inches (0.61 mm), such as about 0.021 inches (0.53 mm). The spline **30** can comprise a substantially rigid material such as, for example, PEEK. The spline can have an inner diameter of about 0.022 inches (0.56 mm) and an outer diameter of about 0.035 inches (0.89 mm). The stylet 50 can have a diameter that is about 0.014 inches (0.36 mm). The inner sleeve 40 can be flexible and comprise a polymer material such as for example, nylon, HDPE or FEP and can have an inner diameter of about 0.024 inches (0.61 mm) and an outer diameter of about 0.028 inches (0.71 mm). The electrode **70** can have an outer diameter of about 0.063 inches (1.6 mm) and an inner diameter that slidably receives the spline. The electrode **75** can have an outer diameter of about 0.105 inches (2.67 mm) and the electrode **76** can have an outer diameter of about 0.084 inches (2.1 mm). The distal end of the lead **10d** can have a diameter of about 0.084 inches (2.1 mm) (with a substantially constant outer diameter from at least about the electrode **76,** and typically from beyond electrode **75** to the tip). Other configurations/sizes and materials for the lead, shaft, spline, sleeve, electrode(s) and stylet may be used. In some embodiments, the stylet is not required. The stylet **50** or another elongate member can be used to facilitate alignment/lateral centering of the two shafts for ease of engagement of the spline(s).

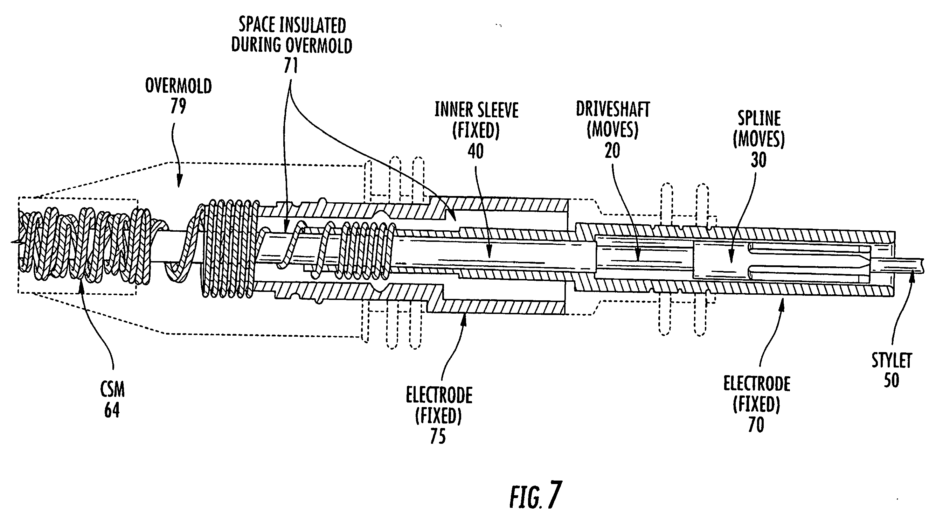

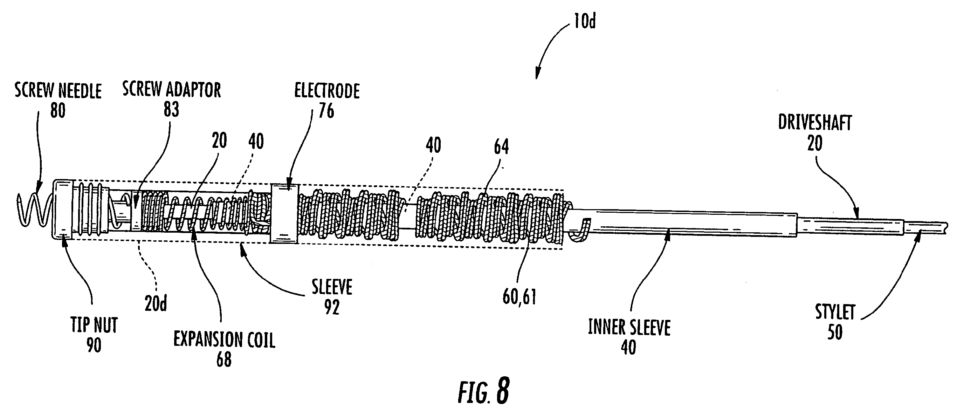

**Figure 7** illustrates that the distal end of the lead **10** can have a tip nut **90** with internal threads that mesh with the screw and that as shown in **Figure 8,** the expansion coil can reside in a silicone or other biocompatible sleeve **92,** which allows the expansion coil to wind up or unwind during extension or retraction of the screw. Similarly, the lead **10** can be encased in a biocompatible material such as slicone overmold **79** as shown in **Figure 7** to have the desired profile shape or size.

In some embodiments, the lead **10** can be a neuromodulation lead or a cardiac lead. The lead can be an implantable lead such as a pacemaker lead. Embodiments of the invention can be particularly suitable for an active fixation bradyarrhythmia lead. The lead can include a distal electrode conductor **61** and/or **62** wound in a two-layer or trilayer CSM **64** along the length of the lead. The proximal electrode conductor **62** can be substantially concentrically arranged outside the distal electrode conductors **60, 61.**

Although the above has primarily described the drive shaft in connection with a lead, the invention is not limited thereto and may be use with any medical device desiring a drive tool. For example, the features of the invention can be used with a catheter, probe or the like.

The foregoing is illustrative of the present invention and is not to be construed as limiting thereof. Although a few exemplary embodiments of this invention have been described, those skilled in the art will readily appreciate that many modifications are possible in the exemplary embodiments without materially departing from the novel teachings and advantages of this invention. Accordingly, all such modifications are intended to be included within the scope of this invention as defined in the claims. The invention is defined by the following claims, with equivalents of the claims to be included therein.

**Claims**

1. A medical device comprising:

   an elongate body (10) having opposing proximal (10p) and distal end (10d) portions with an axially extending center cavity;
   an internal drive shaft (20) residing in the center cavity, the drive shaft having a proximal end portion (20p) with a rotatable spline (30) residing in the proximal end portion of the elongate body; and
   an extendable member (80) held in the distal end portion of the elongate body (10), the extendable member in communication with the drive shaft, whereby rotation of the drive shaft causes the extendable member to extend out of the elongate body, **characterised by** at least one conductor (60) extending along the elongate body, each of the at least one conductor comprising at least one current suppression module (64), each of the at least one current suppression module comprising the conductor turning back on itself twice in a lengthwise direction to from a conductor configuration of a reverse or backward section in one lengthwise direction and proximately located forward sections that extend in an opposing lengthwise direction.

2. A medical device according to claim 1, wherein the elongate body is an intrabody medical lead with at least one electrode with low DC resistance and is flexible.

3. A medical device according to claim 1 or 2, wherein the extendable member comprises a screw electrode.

4. A medical device according to any of claims 1-3, wherein the extendable member is an electrode or sensor in electrical communication with a one of the at least one conductor, and wherein the elongate body is an implantable lead and has a diameter that is less than about 0.10 inches (2.5 mm) over at least a major portion of its length.

5. A medical device according to any of claims 1-4, wherein the elongate body is an implantable neuromodulation lead.

6. A medical device according to any of claims 1-4, wherein the elongate body is an implantable cardiac lead.

7. A medical device according to any of claims 1-4, wherein the elongate body is an implantable pacemaker lead.

8. A medical device according to any of claims 1-7, wherein the elongate body comprises a flexible inner sleeve (40) residing over the drive shaft and at least one conductor portion coiled substantially concentrically about the sleeve.

9. A medical device according to any of claims 1-8, wherein the elongate body comprises a stationary electrode (70) disposed on the proximal end portion of the elongate body about the drive shaft spline.

10. A medical device according to any of claims 1-9, in combination with a single-use disposable drive tool (100), the drive tool having a primary body with an axially extending cavity (101) and a spline and/or spline engagement member (130) residing in a distal end portion of drive the tool primary body (100d), the tool spline and/or spline engagement member adapted to slidably releasably engage the drive shaft spline whereby a user can rotate the drive shaft.

11. A medical device and tool according to claim 10, wherein the drive tool primary body has a bore (102) sized and configured to snugly slidably receive the proximal end portion of the elongate body, wherein the bore has a larger diameter than the axially extending cavity.

12. A medical device and tool according to claim 10, further comprising a stylet (50) that extends out of the proximal end portion (100p) of the drive tool and resides in a center cavity extending through the drive shaft.

13. The medical device according to claim 1, where the elongate body is an implantable pacemaker lead and the drive shaft is an internal rotatable and translatable drive shaft.

14. The medical device according to claim 13, wherein the lead has low DC resistance and is flexible, wherein the extendable member comprises a fixation screw, and wherein the lead is an active fixation bradyarrhythmia lead with a distal electrode.

15. The medical device according to claim 1, further comprising a single-use disposable medical drive tool (100) having an internal spline or spline engagement member sized and configured to slidably releasably receive and engage the rotatable spline residing in the proximal end portion of the elongate body, the drive tool further comprising a cavity (101) for receiving a stylet (50), the drive tool being configured to allow a user to both rotate and translate the internal drive shaft, wherein the medical drive tool is held in a sterile package.

**Patentansprüche**

1. Medizinische Vorrichtung, die aufweist:

einen länglichen Körper (10), der einen proximalen (10p) und distalen (10d) Endabschnitt, die sich gegenüberliegen, und einen sich axial erstreckenden zentralen Hohlraum aufweist;
eine in dem zentralen Hohlraum angeordnete innere Antriebswelle (20), die einen proximalen Endabschnitt (20p) mit einem drehbaren Keil (30) aufweist, der in dem proximalen Endabschnitt des länglichen Körpers angeordnet ist; und
ein in dem distalen Endabschnitt des länglichen Körpers (10) bereitgestelltes ausfahrbares Element (80), das mit der Antriebswelle gekoppelt ist, wobei ein Drehen der Antriebswelle ein Ausfahren des ausfahrbaren Elements aus dem länglichen Körper bewirkt,
**gekennzeichnet durch** mindestens einen sich entlang des länglichen Körpers erstreckenden Leiter (60), wobei jeder des mindestens einen Leiters mindestens ein Stromunterdrückungsmodul (64) aufweist und jedes des mindestens einen Stromunterdrückungsmoduls den in Längsrichtung zweimal auf sich selbst zurückwindenden Leiter aufweist, um eine Leiterkonfiguration zu bilden, die einen Umkehr- oder Rückwärtsabschnitt, der sich in die eine Längsrichtung erstreckt, und angrenzend angeordnete Vorwärtsabschnitte, die sich in die dazu entgegengesetzte Längsrichtung erstrecken, aufweist.

2. Medizinische Vorrichtung nach Anspruch 1, wobei der längliche Körper eine intrakorporale medizinische Zuleitung ist, die mindestens eine Elektrode mit geringem Gleichstrom-Widerstand aufweist, und biegsam ist.

3. Medizinische Vorrichtung nach Anspruch 1 oder 2, wobei das ausfahrbare Element eine Schraubelektrode aufweist.

4. Medizinische Vorrichtung nach einem der Ansprüche 1 bis 3, wobei das ausfahrbare Element eine Elektrode oder ein Sensor in elektrischer Verbindung mit einem des mindestens einen Leiters ist, und wobei der längliche Körper eine implantierbare Zuleitung ist und zumindest über den größten Teil seiner Länge einen Durchmesser hat, der weniger als ungefähr 0,10 Zoll (2,5mm) beträgt.

5. Medizinische Vorrichtung nach einem der Ansprüche 1 bis 4, wobei der längliche Körper eine implantierbare Nervenmodulationszuleitung ist.

6. Medizinische Vorrichtung nach einem der Ansprüche 1 bis 4, wobei der längliche Körper eine implantierbare Herzzuleitung ist.

7. Medizinische Vorrichtung nach einem der Ansprüche 1 bis 4, wobei der längliche Körper eine implantierbare Schrittmacherzuleitung ist.

8. Medizinische Vorrichtung nach einem der Ansprüche 1 bis 7, wobei der längliche Körper eine über der Antriebswelle angeordnete flexible Innenhülse (40) und mindestens einen im Wesentlichen konzentrisch um die Hülse gewickelten Leiterabschnitt aufweist.

9. Medizinische Vorrichtung nach einem der Ansprüche 1 bis 8, wobei der längliche Körper eine stationäre Elektrode (70) aufweist, die an dem proximalen Endabschnitt des länglichen Körpers um den Antriebswellenkeil herum angeordnet ist.

10. Medizinische Vorrichtung nach einem der Ansprüche 1 bis 9 in Kombination mit einem für eine einmalige Verwendung vorgesehenen Antriebswerkzeug (100), wobei das Antriebswerkzeug einen Hauptkörper mit einem sich axial erstreckenden Hohlraum (101) und einen Keil und/oder ein Keileingriffselement (130) aufweist, der und/oder das in einem distalen Endabschnitt (100d) des Hauptkörpers des Antriebswerkzeugs angeordnet ist, wobei der Werkzeugkeil und/oder das Keileingriffselement angepasst sind, gleitend in lösbaren Eingriff mit dem Keil der Antriebswelle gebracht zu werden, um einem Benutzer zu ermöglichen, die Antriebswelle zu drehen.

11. Medizinische Vorrichtung und Werkzeug nach Anspruch 10, wobei der Hauptkörper des Antriebswerkzeugs eine Bohrung (102) hat, die so bemessen und konfiguriert ist, um den proximalen Endabschnitt des länglichen Körpers passgenau gleitend aufzunehmen, wobei die Bohrung (102) einen Durchmesser hat, der größer ist als der Durchmesser des sich axial erstreckenden zentralen Hohlraums.

12. Medizinische Vorrichtung und Werkzeug nach Anspruch 10, ferner mit einem Stilett (50), das aus dem proximalen Endabschnitt (100p) des Antriebswerkzeugs herausragt und in einem sich durch die Antriebswelle erstreckenden Hohlraum untergebracht ist.

13. Medizinische Vorrichtung nach Anspruch 1, wobei der längliche Körper eine implantierbare Schrittmacherzuleitung ist und die Antriebswelle eine drehbare und verschiebbare innere Antriebswelle ist.

14. Medizinische Vorrichtung nach Anspruch 13, wobei die Zuleitung einen geringen Gleichstrom-Widerstand hat und flexibel ist, wobei das ausfahrbare Element eine Fixierschraube aufweist und die Zuleitung eine für aktive Fixation geeignete Bradyarrhythmie-Kabel mit einer distalen Elektrode ist.

**15.** Medizinische Vorrichtung nach Anspruch 1, ferner mit einem für eine einmalige Verwendung bestimmten medizinischen Antriebswerkzeug (100), das einen inneren Keil oder ein inneres Keileingriffselement aufweist, der oder das so bemessen und konfiguriert ist, den im proximalen Endabschnitt des länglichen Körpers angeordneten drehbaren Keil gleitend aufzunehmen und in lösbarem Eingriff zu halten, wobei das Antriebswerkzeug außerdem einen Hohlraum (101) zur Aufnahme eines Stiletts (50) aufweist, wobei das Antriebswerkzeug konfiguriert ist, einem Benutzer sowohl ein Drehen wie auch ein Verschieben der inneren Antriebswelle zu erlauben, wobei das medizinische Antriebswerkzeug in einer sterilen Verpackung bereitgestellt ist.

## Revendications

**1.** Dispositif médical comprenant :

un corps allongé (10) ayant des parties d'extrémité proximale (10p) et d'extrémité distale (10d) opposées avec une cavité centrale s'étendant de manière axiale ;

un arbre d'entraînement interne (20) résidant dans la cavité centrale, l'arbre d'entraînement ayant une partie d'extrémité proximale (20p) avec une cannelure rotative (30) résidant dans la partie d'extrémité proximale du corps allongé ; et

un élément extensible (80) maintenu dans la partie d'extrémité distale du corps allongé (10), l'élément extensible étant en communication avec l'arbre d'entraînement, moyennant quoi la rotation de l'arbre d'entraînement amène l'élément extensible à s'étendre hors du corps allongé, **caractérisé par** au moins un conducteur (60) s'étendant le long du corps allongé, chaque au moins un conducteur comprenant au moins un module de suppression de courant (64), chaque au moins un module de suppression de courant comprenant le conducteur se retournant sur lui-même deux fois dans le sens de la longueur afin de former une configuration de conducteur d'une section opposée ou arrière dans un sens de la longueur et des sections avant positionnées de manière proximale qui s'étendent dans un sens de la longueur opposé.

**2.** Dispositif médical selon la revendication 1, dans lequel le corps allongé est un fil médical intracorporel avec au moins une électrode avec une faible résistance CC et est souple.

**3.** Dispositif médical selon la revendication 1 ou 2, dans lequel l'élément extensible comprend une électrode à vis.

**4.** Dispositif médical selon l'une quelconque des revendications 1 à 3, dans lequel l'élément extensible est une électrode ou un capteur en communication électrique avec l'un du au moins un conducteur, et dans lequel le corps allongé est un fil implantable et a un diamètre qui est inférieur à environ 0,10 pouces (2,5 mm) sur au moins une majeure partie de sa longueur.

**5.** Dispositif médical selon l'une quelconque des revendications 1 à 4, dans lequel le corps allongé est un fil de neuromodulation implantable.

**6.** Dispositif médical selon l'une quelconque des revendications 1 à 4, dans lequel le corps allongé est un fil cardiaque implantable.

**7.** Dispositif médical selon l'une quelconque des revendications 1 à 4, dans lequel le corps allongé est un fil de stimulateur cardiaque implantable.

**8.** Dispositif médical selon l'une quelconque des revendications 1 à 7, dans lequel le corps allongé comprend un manchon interne souple (40) résidant sur l'arbre d'entraînement et au moins une partie de conducteur enroulée de manière sensiblement concentrique autour du manchon.

**9.** Dispositif médical selon l'une quelconque des revendications 1 à 8, dans lequel le corps allongé comprend une électrode fixe (70) disposée sur la partie d'extrémité proximale du corps allongé autour de la cannelure d'arbre d'entraînement.

**10.** Dispositif médical selon l'une quelconque des revendications 1 à 9, en combinaison avec un outil d'entraînement jetable à usage unique (100), l'outil d'entraînement ayant un corps principal avec une cavité (101) s'étendant de manière axiale et une cannelure et/ou un élément de mise en prise de cannelure (130) résidant dans une partie d'extrémité distale du corps principal (100d) de l'outil d'entraînement, la cannelure d'outil et/ou l'élément de mise en prise de cannelure étant adapté(e) pour mettre en prise de manière coulissante et amovible la cannelure d'arbre d'entraînement, moyennant quoi l'utilisateur peut faire tourner l'arbre d'entraînement.

11. Dispositif médical et outil selon la revendication 10, dans lesquels le corps principal d'outil d'entraînement a un alésage (102) dimensionné et configuré pour recevoir de manière coulissante à frottement doux la partie d'extrémité proximale du corps allongé, dans lequel l'alésage a un plus grand diamètre que la cavité s'étendant de manière axiale.

12. Dispositif médical et outil selon la revendication 10, comprenant en outre un stylet (50) qui s'étend hors de la partie d'extrémité proximale (100p) de l'outil d'entraînement et se trouve dans une cavité centrale s'étendant à travers l'arbre d'entraînement.

13. Dispositif médical selon la revendication 1, dans lequel le corps allongé est un fil de stimulateur cardiaque implantable et l'arbre d'entraînement est un arbre d'entraînement rotatif interne et pouvant effectuer une translation.

14. Dispositif médical selon la revendication 13, dans lequel le fil a une faible résistance CC et est souple, dans lequel l'élément extensible comprend une vis de fixation et dans lequel le fil est un fil de bradyarythmie à fixation active avec une électrode distale.

15. Dispositif médical selon la revendication 1, comprenant en outre un outil d'entraînement médical jetable à usage unique (100) ayant une cannelure interne ou élément de mise en prise de cannelure dimensionné(e) et configuré(e) pour recevoir et mettre en prise de manière coulissante et amovible la cannelure rotative résidant dans la partie d'extrémité proximale du corps allongé, l'outil d'entraînement comprenant en outre une cavité (101) pour recevoir un stylet (50), l'outil d'entraînement étant configuré pour permettre à un utilisateur à la fois de faire tourner et de faire faire un mouvement de translation à l'arbre d'entraînement interne, dans lequel l'outil d'entraînement médical est contenu dans un emballage stérile.

FIG. 1

STYLET
50

ELECTRODE
70

ELECTRODE
75

ELECTRODE
76

60,61

65

62

64

68

TIP,
BODY

83

20d

FIG. 2

EP 2 265 323 B1

SPACE INSULATED
DURING OVERMOLD
71

INNER TUBULAR
SLEEVE (FIXED)
40

DRIVESHAFT
(MOVES)
20

SPLINE
(MOVES)
30

79

60,61

62

71

CSM
64

40

60

ELECTRODE
(FIXED)
75

PROXIMAL
ELECTRODE
(FIXED)
NON-ROTATING
70

STYLET
50

FIG. 3

EP 2 265 323 B1

*FIG. 4A*

*FIG. 4B*

FIG. 5A

FIG. 5B

EP 2 265 323 B1

*FIG. 6*

STYLET
50

SPLINE
30

10p

DRIVESHAFT
20

10

ELECTRODE
70

INNER TUBULAR
SLEEVE
40

60

61

FIG. 7

SCREW NEEDLE
80

SCREW ADAPTOR
83

20  40

ELECTRODE
76

10d

40  64

DRIVESHAFT
20

TIP NUT
90

20d

EXPANSION COIL
68

SLEEVE
92

60,61

INNER SLEEVE
40

STYLET
50

FIG. 8

EP 2 265 323 B1

**EP 2 265 323 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2003204232 A1
- US 955724 P
- US 12047832 B